# EUROPEAN PATENT APPLICATION

(11) **EP 1 284 292 A2**
(43) Date of publication of application: **19.02.2003**
(21) Application number: 02021846.7
(22) Date of filing: 17.03.1995
(51) Int. Cl.: C12N 15/12, C12N 15/63, C07K 14/82, C07K 16/18, A61K 48/00, A61K 38/17, C12Q 1/68, G01N 33/53, G01N 33/68

(54) **Human B-cell Translocation Gene-3**

(62) Divisional of application: 95913736.5
(71) Applicant: HUMAN GENOME SCIENCES, INC., Rockville, MD 20850-3338 (US)
(72) Inventor: The designation of the inventor has not yet been filed
(74) Representative: VOSSIUS & PARTNER

(57) **Abstract**

Human B-cell translocation gene-3 and polypeptides encoded by such genes and a procedure for producing such polypeptides by recombinant techniques is disclosed. Also disclosed are methods for utilizing such polypeptide for the treatment of aberrant cellular proliferation. Also disclosed are diagnostic assays for detecting susceptibility to diseases associated with mutations in the nucleic acid sequences encoding said polypeptides and for detecting altered levels of such polypeptides.

## Description

This invention relates to newly identified polynucleotides, polypeptides encoded by such polynucleotides, the use of such polynucleotides and polypeptides, as well as the production of such polynucleotides and polypeptides. The polypeptides of the present invention have been putatively identified as human B-cell translocation genes-2 and 3, hereinafter referred to as "ETG-2 and BTG-3". The invention also relates to inhibiting the action of such polypeptides.

In normal tissues, homeostasis is maintained through negative and positive growth controls which effect the proliferation and differentiation related to cellular genetic programs. An alteration of this subtle balance can result in developmental abnormalities or in neoplasia. Proto-oncogenes, genes that promote cell division, were the first growth-inducing elements to be identified and more than sixty of them have been described so far (Bishop, J.M., Cell, 64:235:248 (1991)). The genes that negatively regulate cell proliferation are crucial to counteract the growth-inducing elements and are likely to have the same importance as proto-oncogenes in controlling cell division (Marshall, C.J., Cell, 64:313-326 (1991), especially since the loss of their function has been reported to be associated with irregular cellular differentiation and proliferation or with alteration of embryonic development (Weinberg, R.A., Science, 254:1138-1146 (1991)) .

The polynucleotides and polypeptides of the present invention are thought to be members of a family of anti-proliferative genes. BTG-1 is a member of this group and has been cloned and expressed. (Rovault, J.P., *et al*., The EMBO Journal, 11(4):1663-1670 (1992). BTG-1 was shown to negatively regulate NlH3T3 cell proliferation when over- or inappropriately expressed. BTG stands for B-cell translocation gene, and the BTG-1 gene has been shown to be involved in a chromosomal translocation [t(8;12) (q24;22)] in B-cell chronic lymphocytic leukemia.

The BTG-1 open reading frame is 60% homologous to PC3, an immediate early gene induced by nerve growth factor in rat PC12 cells. Sequence and Northern blot analyses indicate that BTG-1 and PC3 are not cognate genes but are thought to be members of this new family of anti-proliferation genes. The BTG-1 gene is preferentially expressed in quiescent cells during the early sub-phases of G₁ in a serum-dependent manner and it is then down-regulated to reach a minimum level as the cells enter the S phase. This suggests a functional link between BTG-1 and the cell cycle process. BTG-1 is expressed in tissues (lymphoid, liver, placenta) containing non-dividing cells likely to re-enter the cell cycle upon different stimuli, whereas the expression of BTG-1 is barely detectable in fully differentiated tissues such as brain and muscle.

The BTG-1 gene was shown to be highly conserved in evolution and a similar 1.8 Kb transcript can be detected in murine and chicken tissue by using a human BTG-1 DNA probe (Rimokh, R. *et al*., Genes Chrom. Cancer, 3:24-36 (1991)).

The BTG-2 and BTG-3 genes and gene products have been putatively identified as members of this family as a result of amino acid sequence homology to BTG-1.

The term "gene" or "cistron" means the segment of DNA involved in producing a polypeptide chain; it includes regions preceding and following the coding region (leader and trailer) as well as intervening sequences (introns) between individual coding segments (exons).

In accordance with one aspect of the present invention, there are provided novel mature polypeptides which have been putatively identified as BTG-2 and BTG-3, as well as biologically active and diagnostically or therapeutically useful fragments, analogs and derivatives thereof.

In accordance with another aspect of the present invention, there are provided isolated nucleic acid molecules encoding the polypeptides of the present invention, including mRNAs, DNAs, cDNAs, genomic DNAs as well as analogs and biologically active and diagnostically or therapeutically useful fragments and derivatives thereof.

In accordance with yet a further aspect of the present invention, there are also provided nucleic acid probes comprising nucleic acid molecules of sufficient length to specifically hybridize to the gene sequences of the present invention.

In accordance with yet a further aspect of the present invention, there is provided a process for producing such polypeptides by recombinant techniques, comprising culturing recombinant prokaryotic and/or eukaryotic host cells, containing nucleic acid sequences which encode for the polypeptides of the present invention, under conditions promoting expression of said protein and subsequent recovery of said protein.

In accordance with yet a further aspect of the present invention, there is provided a process for utilizing such polypeptides, or polynucleotides encoding such polypeptides for therapeutic purposes, for example, to treat disease states characterized by aberrant cellular proliferation, and to modulate cellular growth.

In accordance with yet a further aspect of the present invention, there are provided antibodies against such polypeptides.

In accordance with yet another aspect of the present invention, there are provided antagonists to such polypeptides, which may be used to inhibit the action of such polypeptides, for example, in the treatment of diseases related to chromosomal translocation, for example, lymphocytic leukemia.

In accordance with still another aspect of the present invention, there are provided diagnostic assays for detecting diseases related to the under-expression of the polypeptides of the present invention and mutations in the nucleic acid sequences encoding such polypeptides.

In accordance with yet a further aspect of the present invention, there is provided a process for utilizing such polypeptides, or polynucleotides encoding such polypeptides, for *in vitro* purposes related to scientific research, synthesis of DNA and manufacture of DNA vectors.

These and other aspects of the present invention should be apparent to those skilled in the art from the teachings herein.

The following drawings are illustrative of embodiments of the invention and are not meant to limit the scope of the invention as encompassed by the claims.

Figure 1 is an illustration of the cDNA and corresponding deduced amino acid sequence the putative BTG-2. One-letter abbreviations for amino acids are used. Sequencing was performed, for both BTG-2 and BTG-3, using a 373 Automated DNA sequencer (Applied Biosystems, Inc.). Sequencing accuracy is predicted to be greater than 97% accurate.

Figure 2 shows the cDNA and corresponding deduced amino acid sequence of the putative BTG-3.

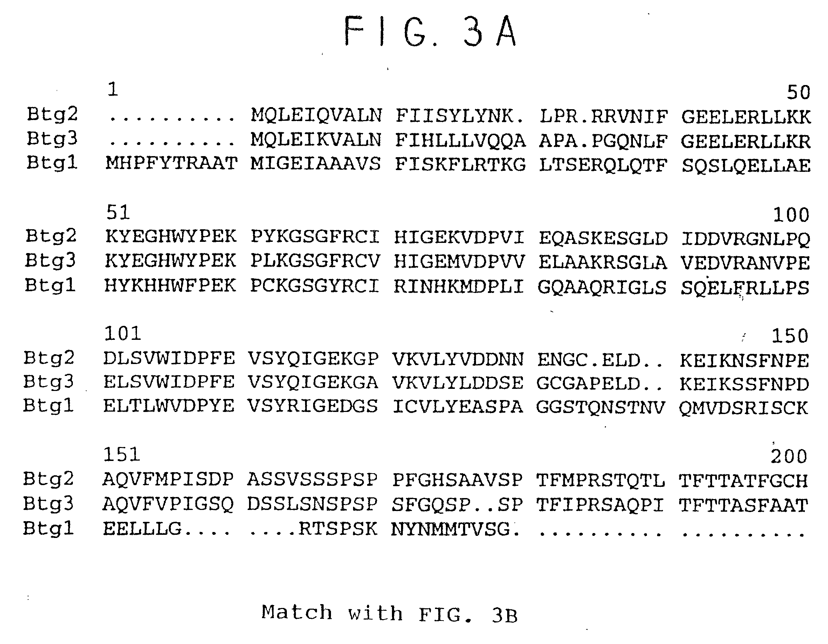

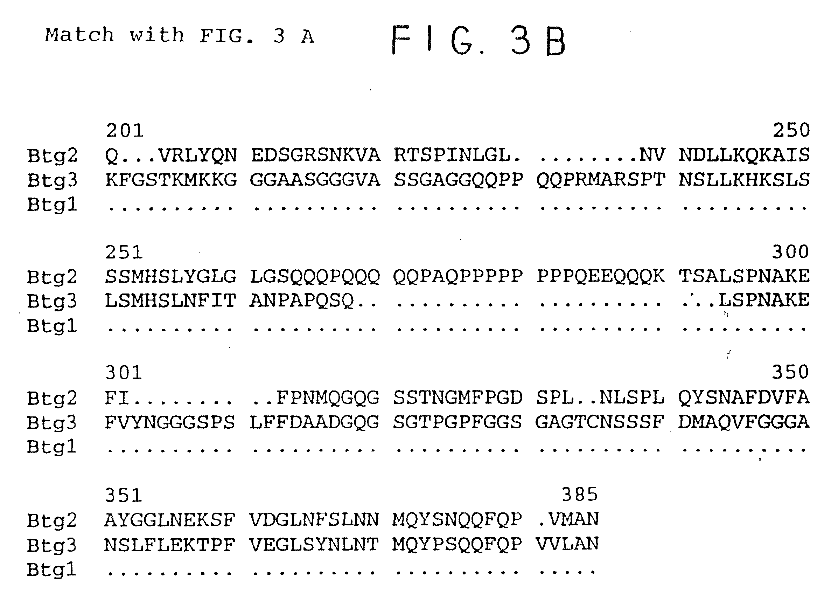

Figure 3 is an amino acid sequence alignment between BTG-1, BTG-2 and BTG-3 proteins, wherein m represents mouse and h represents human.

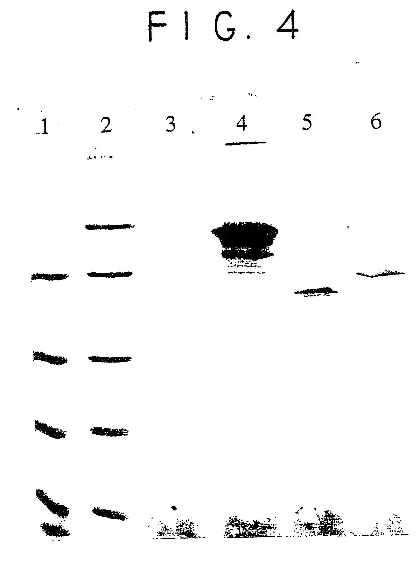

Figure 4 is a photograph of a gel after *in vitro* translation and electrophoresis of BTG-2 and BTG-3. Lane 1: Low molecular weight markers; Lane 2: High molecular weight markers; Lane 3: Blank; Lane 4: T3; Lane 5: BTG-3; Lane 6: BTG-2.

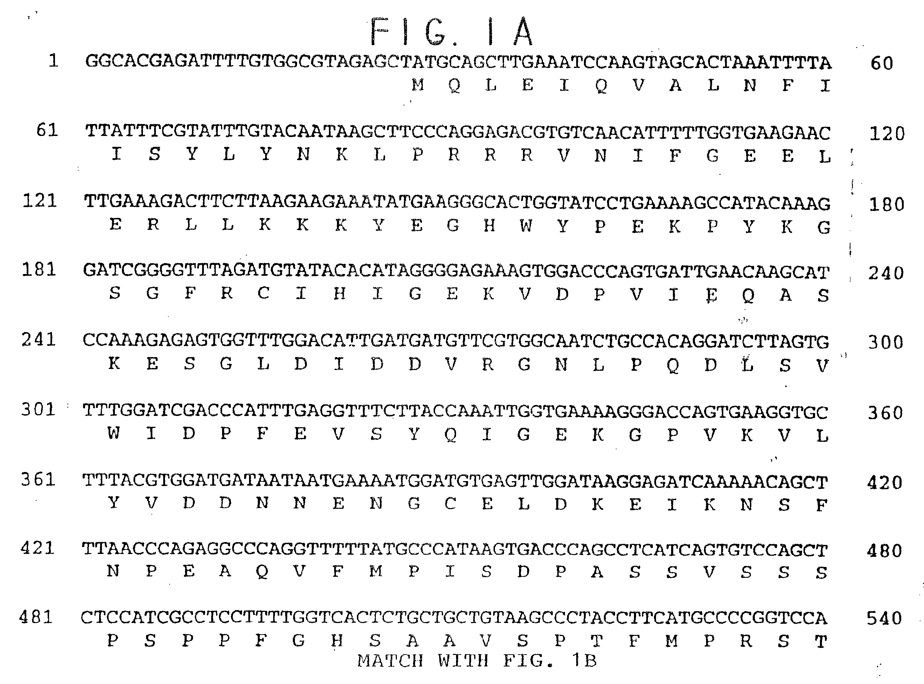

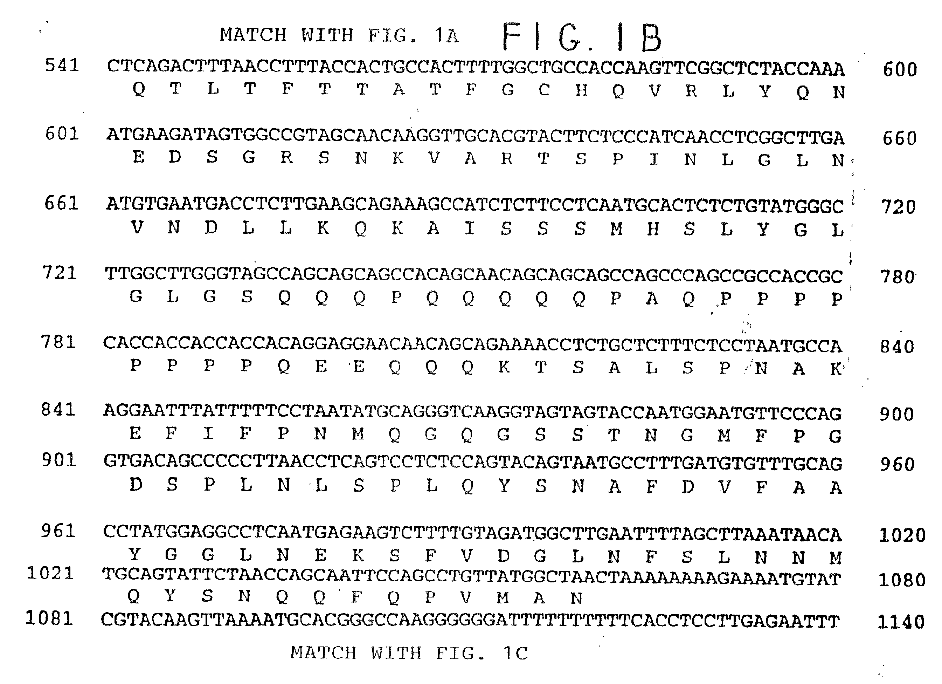

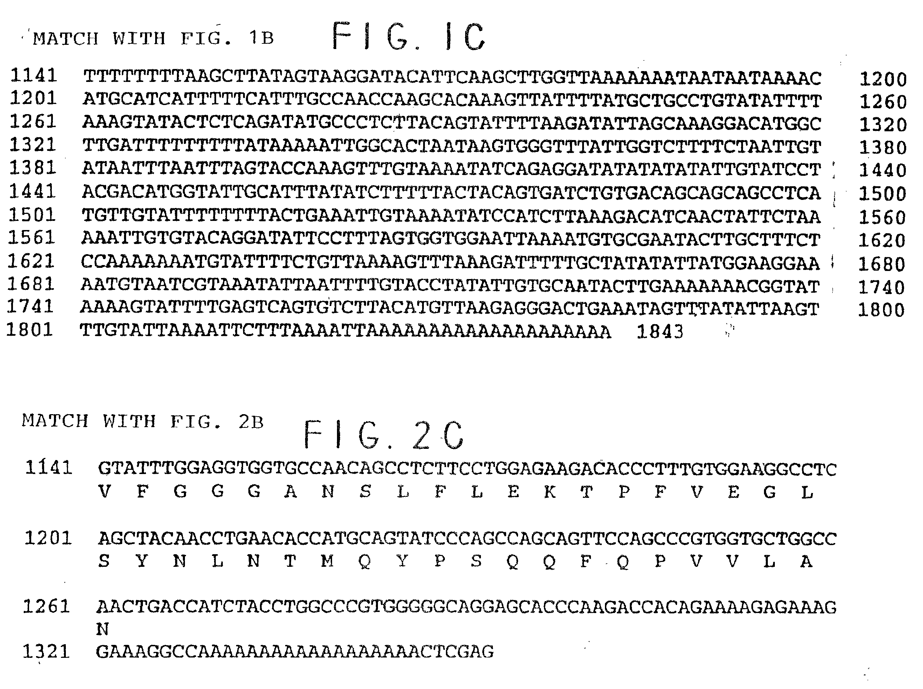

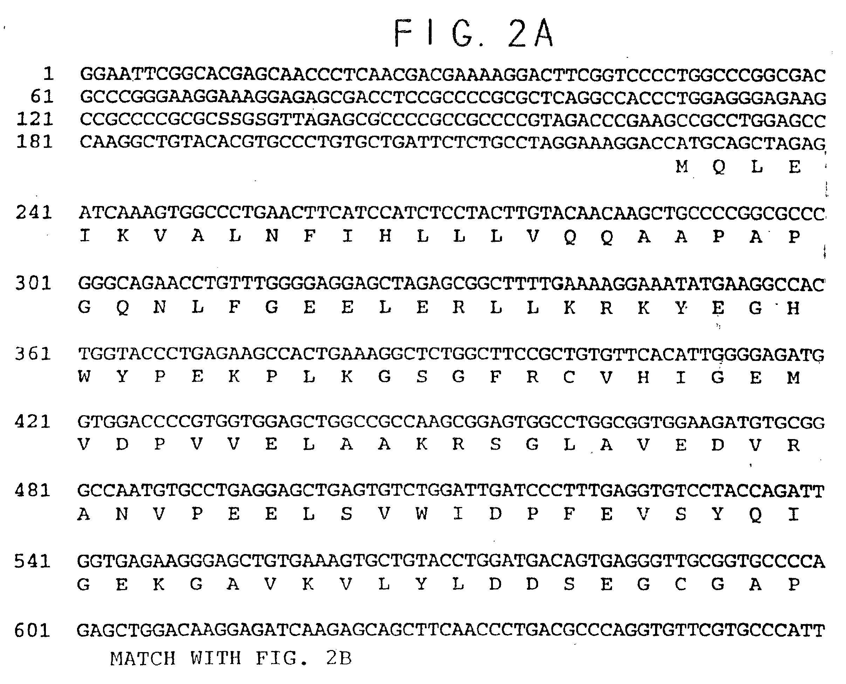

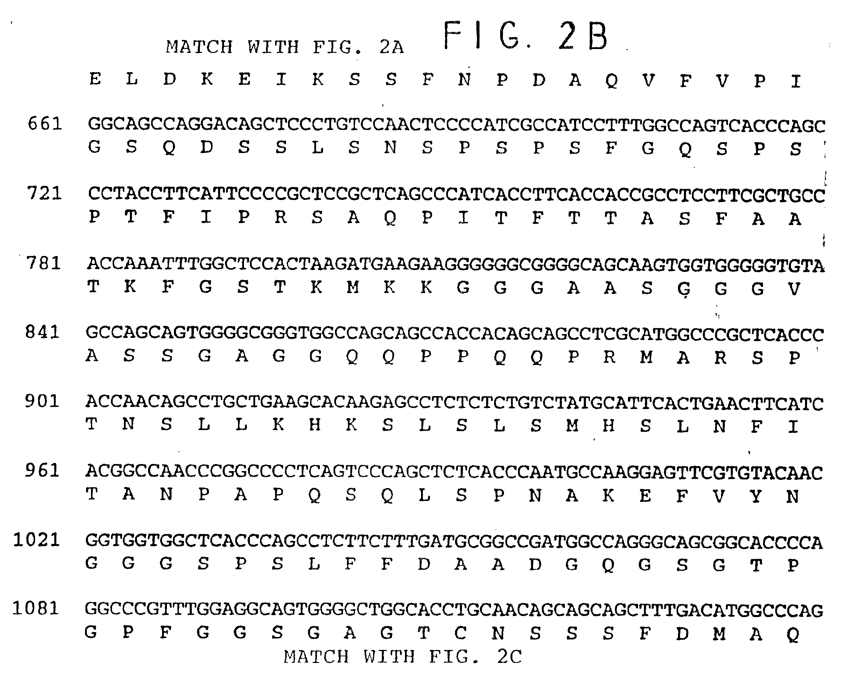

In accordance with an aspect of the present invention, there are provided isolated nucleic acids (polynucleotides) which encode for the mature polypeptides having the deduced amino acid sequences of Figures 1 and 2 (SEQ ID No. 2 and 4) or for the mature polypeptides encoded by the cDNAs of the clone(s) deposited as ATCC Deposit No. 97025 deposited January 18, 1995 for BTG-2 and ATCC No. 97010 deposited on January 5, 1995 for BTG-3.

A polynucleotide encoding the BTG-2 polypeptide of the present invention may be obtained from liver, lymphoid tissue and placenta. The polynucleotide of this invention was discovered in a cDNA library derived from a human endometrial tumor. It is structurally related to BTG-1. It contains an open reading frame encoding a protein of 345 amino acid residues of which approximately the first 25 amino acids residues are the putative leader sequence such that the mature protein comprises 320 amino acids. The protein exhibits the highest degree of homology to BTG-1 with 49 % identity and 72 % similarity over a 91 amino acid stretch.

A polynucleotide encoding a BTG-3 polypeptide of the present invention may be obtained from synovial sarcoma, cerebellum, embryonic tissues and placenta. The polynucleotide of this invention was discovered in a cDNA library derived from human synovial carcinoma. It is structurally related to the BTG family. It contains an open reading frame encoding a protein of 345 amino acid residues of which approximately the first 25 amino acids residues are the putative leader sequence such that the mature protein comprises 320 amino acids. The protein exhibits the highest degree of homology to BTG-1 with 48 % identity and 74 % similarity over an 85 amino acid stretch. Over the same stretch of 85 amino acids, BTG-2 and BTG-3 are 83 % identical and 87 % similar to each other. In addition, BTG-2 is approximately 143 amino acids longer at the carboxy terminus as compared to BTG-1, while BTG-3 is approximately 162 amino acids longer at the carboxy terminus.

The polynucleotides of the present invention may be in the form of RNA or in the form of DNA, which DNA includes cDNA, genomic DNA, and synthetic DNA. The DNA may be doublestranded or single-stranded, and if single stranded may be the coding strand or non-coding (anti-sense) strand. The coding sequence which encodes the mature polypeptide may be identical to the coding sequence shown in Figures 1 and 2 (SEQ ID No. 1 and 3) or that of the deposited clone or may be a different coding sequence which coding sequence, as a result of the redundancy or degeneracy of the genetic code, encodes the same mature polypeptides as the DNA of Figures 1 and 2 (SEQ ID No. 1 and 3) or the deposited cDNA.

The polynucleotides which encodes for the mature polypeptide of Figures 1 and 2 (SEQ ID No. 2 and 4) or for the mature polypeptides encoded by the deposited cDNA may include: only the coding sequence for the mature polypeptide; the coding sequence for the mature polypeptide (and optionally additional coding sequence) and non-coding sequence, such as introns or non-coding sequence 5' and/or 3' of the coding sequence for the mature polypeptide.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptides having the deduced amino acid sequences of Figures 1 and 2 (SEQ ID No. 2 and 4) or the polypeptides encoded by the cDNAs of the deposited clones. The variants of the polynucleotides may be a naturally occurring allelic variant of the polynucleotides or a non-naturally occurring variant of the polynucleotides.

Thus, the present invention includes polynucleotides encoding the same mature polypeptides as shown in Figures 1 and 2 (SEQ ID No. 2 and 4) or the same mature polypeptides encoded by the cDNAs of the deposited clones as well as variants of such polynucleotides which variants encode for a fragment, derivative or analbg of the polypeptides of Figures 1 and 2 (SEQ ID No. 2 and 4) or the polypeptides encoded by the CDNAs of the deposited clones. Such nucleotide variants include deletion variants, substitution variants and addition or insertion variants.

As hereinabove indicated, the polynucleotides may have a coding sequence which is a naturally occurring allelic variant of the coding sequences shown in Figures 1 and 2 (SEQ ID No. 1 and 3) or of the coding sequence of the deposited clones. As known in the art, an allelic variant is an alternate form of a polynucleotide sequence which may have a substitution, deletion or addition of one or more nucleotides, which does not substantially alter the function of the encoded polypeptides.

The polynucleotides of the present invention may also have the coding sequence fused in frame to a marker sequence which allows for purification of the polypeptides of the present invention. The marker sequence may be a hexa-histidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The present invention further relates to polynucleotides which hybridize to the hereinabove-described sequences if there is at least 50% and preferably 70% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which retain substantially the same biological function or activity as the mature polypeptides encoded by the cDNAs of Figures 1 and 2 (SEQ ID No. 1 and 3) or the deposited cDNAs.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to polypeptides which have the deduced amino acid sequences of Figures 1 and 2 (SEQ ID No. 2 and 4) or which have the amino acid sequences encoded by the deposited cDNAs, as well as fragments, analogs and derivatives of such polypeptides.

The terms "fragment," "derivative" and "analog" when referring to the polypeptides of Figures 1 and 2 (SEQ ID No. 2 and 4) or that encoded by the deposited cDNAs, means polypeptides which retain essentially the same biological function or activity as such polypeptides. Thus, an analog includes a proprotein which can be activated by cleavage of the proprotein portion to produce an active mature polypeptide.

The polypeptides of the present invention may be a recombinant polypeptide, a natural polypeptide or a synthetic polypeptide, preferably a recombinant polypeptide.

The fragment, derivative or analog of the polypeptides of Figures 1 and 2 (SEQ ID No. 2 and 4) or that encoded by the deposited cDNAs may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptides are fused with another compound, such as a compound to increase the half-life of the polypeptide (for example, polyethylene glycol), or (iv) one in which the additional amino acids are fused to the mature polypeptide. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the genes of the present invention. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Spodoptera Sf9; animal cells such as CHO, HEK, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen) , pBS, pD10, phagescript, psiX174, pbluescript SK, pbsks, pNH8A, pNH16a, pNH18A, pNH46A (Stratagene); ptrc99a, pKK223-3, pKK233-3, pDR540, pRIT5 (Pharmacia). Eukaryotic: pWLNEO, pSV2CAT, pOG44, pXT1 pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are PKK232-8 and PCM7. Particular named bacterial promoters include lacI, lacZ, T3, T7, gpt, lambda P_{R}, P_{L} and trp. Eukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene product encoded by the recombinant sequence. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples including the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation, initiation and termination sequences. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and GEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:175 (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptides of the present invention can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

Fragments of the full length genes of the present invention may be used as a hybridization probe for a cDNA library to isolate other genes which have a high sequence similarity to the gene or similar biological activity. Probes of this type generally have at least 50 bases, although they may have a greater number of bases. The probe may also be used to identify a cDNA clone corresponding to a full length transcript and a genomic clone or clones that contain the complete gene including regulatory and promotor regions, exons, and introns. As an example of a screen comprises isolating the coding region of the genes by using the known DNA sequence to synthesize an oligonucleotide probe. Labeled oligonucleotides having a sequence complementary to that of the genes of the present invention are used to screen a library of human cDNA, genomic DNA or mRNA to determine which members of the library the probe hybridizes to.

The BTG-2 and BTG-3 polypeptides have an anti-proliferative ability and may be employed to treat diseases or pathological conditions associated with aberrant cellular proliferation. The polypeptides may be employed as a tumor/growth suppression regulator. They may also be employed to inhibit cancer cell proliferation.

BTG-2 and BTG-3 may also be employed to prevent uncontrolled wound healing which would otherwise cause scarring. Restenosis, which is re-occlusion of arterial walls after balloon angioplasty, may also be treated with BTG-2 and BTG-3 since arteries re-occlude through cell proliferation. Similarly angiogenesis of tumors may be inhibited.

The BTG-2 and BTG-3 genes and gene products may also be employed for modulation of cellular growth. Due to their anti-proliferative effect they could be selectively administered or possibly inhibited when it is desirable to have certain cells proliferate. An example would be a disorder related to the underproduction of certain cells, where proliferation and differentiation of these cells would help to treat the disorder.

The polynucleotides and polypeptides encoded by such polynucleotides may also be utilized for *in vitro* purposes related to scientific research, synthesis of DNA and manufacture of DNA vectors and for designing therapeutics and diagnostics for the treatment of human disease.

The nucleic acid sequences of the present invention may be employed as part of a diagnostic assay for detecting susceptibility to diseases associated with aberrant cellular proliferation. Since, the polypeptides of the present invention are anti-proliferative genes, a disruption in the transcription of the genes and corresponding lack of production of the gene product will likely be involved in aberrant cellular proliferation associated with a malignant phenotype.

Individuals carrying mutations in the genes may be detected at the DNA level by a variety of techniques. Nucleic acids for diagnosis may be obtained from a patient's cells, including but not limited to blood, urine, saliva, tissue biopsy and autopsy material. The genomic DNA may be used directly for detection or may be amplified enzymatically by using PCR (Saiki *et al*., Nature, 324:163-166 (1986)) prior to analysis. RNA or cDNA may also be used for the same purpose. As an example, PCR primers complementary to the nucleic acid encoding the polypeptides of the present invention can be used to identify and analyze mutations. For example, deletions and insertions can be detected by a change in size of the amplified product in comparison to the normal genotype. Point mutations can be identified by hybridizing amplified DNA to radiolabeled RNA of the present invention or alternatively, radiolabeled antisense DNA sequences. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al*., Science, 230:1242 (1985)).

Sequence changes at specific locations may also be revealed by nuclease protection assays, such as RNase and S1 protection or the chemical cleavage method (e.g., Cotton *et al*., PNAS, USA, 85:4397-4401 (1985)) .

Thus, the detection of a specific DNA sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA.

In addition to more conventional gel-electrophoresis and DNA sequencing, mutations can also be detected by *in situ* analysis.

The present invention also relates to a diagnostic assay for detecting altered levels of the proteins of the present invention in various tissues since an over-expression of the proteins compared to normal control tissue samples may detect the presence of a disease or susceptibility to a disease, for example, abnormal cellular proliferation and differentiation. Assays used to detect levels of these proteins in a sample derived from a host are well-known to those of skill in the art and include radioimmunoassays, competitive-binding assays, Western Blot analysis, ELISA assays and "sandwich" assay. An ELISA assay (Coligan, et al., Current Protocols in Immunology, 1(2), Chapter 6, (1991)) initially comprises preparing an antibody specific to the antigens to the polypeptides of the present invention, preferably a monoclonal antibody. In addition a reporter antibody is prepared against the monoclonal antibody. To the reporter antibody is attached a detectable reagent such as radioactivity, fluorescence or, in this example, a horseradish peroxidase enzyme. A sample is removed from a host and incubated on a solid support, e.g. a polystyrene dish, that binds the proteins in the sample. Any free protein binding sites on the dish are then covered by incubating with a non-specific protein, for example, bovine serum albumen. Next, the monoclonal antibody specific to the polypeptides of the present invention is incubated in the dish during which time the monoclonal antibodies attach to any proteins attached to the polystyrene dish. All unbound monoclonal antibody is washed out with buffer. The reporter antibody linked to horseradish peroxidase is now placed in the dish resulting in binding of the reporter antibody to any monoclonal antibody bound to the proteins of the present invention. Unattached reporter antibody is then washed out. Peroxidase substrates are then added to the dish and the amount of color developed in a given time period is a measurement of the amount of protein present in a given volume of patient sample when compared against a standard curve.

A competition assay may be employed wherein antibodies specific to the polypeptides of the present invention are attached to a solid support. Labeled polypeptides and a sample derived from the host are then passed over the solid support and the amount of label- detected, for example by liquid scintillation chromatography, can be correlated to a quantity of the polypeptides of the present invention in the sample.

A "sandwich" assay is similar to an ELISA assay. In a "sandwich" assay the polypeptides of the present invention are passed over a solid support and bind to antibodies attached to a solid support. A second antibody is then bound to the polypeptides. A third antibody which is labeled and specific to the second antibody is then passed over the solid support and binds to the second antibody and an amount can then be quantified.

This invention provides a method for identification of the receptors for the polypeptides of the present invention. The gene encoding the receptor can be identified by numerous methods known to those of skill in the art, for example, ligand panning and FACS sorting (Coligan, et al., Current Protocols in Immun., 1(2), Chapter 5, (1991)). Preferably, expression cloning is employed wherein polyadenylated RNA is prepared from a cell responsive to the polypeptides, and a cDNA library created from this RNA is divided into pools and used to transfect COS cells or other cells that are not responsive to the polypeptides. Transfected cells which are grown on glass slides are exposed to the labeled polypeptides. The polypeptides can be labeled by a variety of means including iodination or inclusion of a recognition site for a site-specific protein kinase. Following fixation and incubation, the slides are subjected to auto-radiographic analysis. Positive pools are identified and sub-pools are prepared and re-transfected using an iterative sub-pooling and re-screening process, eventually yielding a single clones that encodes the putative receptor.

As an alternative approach for receptor identification, the labeled polypeptides can be photo-affinity linked with cell membrane or extract preparations that express the receptor molecule. Cross-linked material is resolved by PAGE analysis and exposed to X-ray film. The labeled complex containing the receptors of the polypeptides can be excised, resolved into peptide fragments, and subjected to protein micro-sequencing. The amino acid sequence obtained from micro-sequencing would be used to design a set of degenerate oligonucleotide probes to screen a cDNA library to identify the genes encoding the putative receptors.

The present invention further provides a method of identifying agonist and antagonist compounds to the gene and gene products of the present invention. An example of such an assay comprises contacting a mammalian cell or membrane preparation expressing the receptors of the polypeptides with labeled polypeptides, eg. by radioactivity, in the presence of a compound to be screened. The ability of the compound to block and enhance the interaction of the polypeptides of the present invention with its receptor is then measured, for example, by liquid scintillation chromatography.

This invention provides a method of screening drugs to identify those which enhance or inhibit interaction of the polypeptides with their receptors. As an example, a mammalian cell or membrane preparation expressing the receptor would be incubated with labeled polypeptides in the presence of the drug. The ability of the drug to enhance or block this interaction could then be measured.

Alternatively, the response of a known second messenger system following interaction of the polypeptides and their receptors would be measured and compared in the presence or absence of the drug. Such second messenger systems include but are not limited to, cAMP guanylate cyclase, ion channels or phosphoinositide hydrolysis.

Potential antagonists to BTG-2 or BTG-3 polypeptides include an antibody, or in some cases, an oligopeptide, which binds to the polypeptide. Alternatively, a potential antagonist may be a closely related protein which binds to the receptor sites, however, they are inactive forms of the polypeptide and thereby prevent their action since receptor sites are occupied.

Another potential antagonist is an antisense construct prepared using antisense technology. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple helix - see Lee et al., Nucl. Acids Res., 6:3073 (1979); Cooney et al, Science, 241:456 (1988); and Dervan et al., Science, 251: 1360 (1991)), thereby preventing transcription and the production of BTG-2 and/or BTG-3. The antisense RNA oligonucleotide hybridizes to the mRNA *in vivo* and blocks translation of the mRNA molecule into the polypeptides (Antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of the polypeptides of the present invention.

Potential antagonists also include a small molecule which binds to and occupies the active site of the polypeptides thereby making them inaccessible to substrate such that normal biological activity is prevented. Examples of small molecules include but are not limited to small peptides or peptide-like molecules.

The antagonists may be employed to treat leukemia, which results from oncogene activation in hemopoietic cells due to a chromosomal translocation. The polypeptides of the present invention may have a direct or indirect function in the activation of a cellular oncogene resulting in leukemia.

The antagonists may be employed in a composition with a pharmaceutically acceptable carrier, e.g., as hereinafter described.

The polypeptides and agonists or antagonists of the present invention may be employed in combination with a suitable pharmaceutical carrier. Such compositions comprise a therapeutically effective amount of the polypeptide, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides and agonists or antagonists of the present invention may be employed in conjunction with other therapeutic compounds.

The pharmaceutical compositions may be administered in a convenient manner such as by the topical, intravenous, intramuscular or subcutaneous routes. The pharmaceutical compositions are administered in an amount which is effective for treating and/or prophylaxis of the specific indication. In general, they are administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The polypeptides of the present invention, and agonists and antagonists which are polypeptides, may also be employed in accordance with the present invention by expression of such polypeptides *in vivo*, which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clones in an analogous manner. Other mapping strategies that can similarly be used to map to its chromosome include *in* situ hybridization, prescreening with labeled flow-sorted chromosomes and preselection by hybridization to construct chromosome specific-cDNA libraries.

Fluorescence *in situ* hybridization (FISH) of a cDNA clone to a metaphase chromosomal spread can be used to provide a precise chromosomal location in one step. This technique can be used with cDNA as short as 500 or 600 bases; however, clones larger than 2,000 bp have a higher likelihood of binding to a unique chromosomal location with sufficient signal intensity for simple detection. For example, 2,000 bp is good, 4,000 is better, and more than 4,000 is probably not necessary to get good results a reasonable percentage of the time. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units of T4 DNA ligase ("ligase") per 0.5 µg of approximately equimolar amounts of the DNA fragments to be ligated.

Unless otherwise stated, transformation was performed as described in the method of Graham, F. and Van der Eb, A., Virology, 52:456-457 (1973):

### Example 1

### Cloning and expression of BTG-2 using the baculovirus expression system

The DNA sequence encoding the full length BTG-2 protein, ATCC # 97025, is amplified using PCR oligonucleotide primers corresponding to the 5' and 3' end sequences of the gene:

The 5' primer has the sequence 5' CAGTGGATCCGCCAC CATGCAGCTTGAAATCCAAGTAGCAC 3' (SEQ ID No. 5) and contains a Bam HI restriction enzyme site (in bold) followed by 6 nucleotides resembling an efficient signal for the initiation of translation in eukaryotic cells (Kozak, M., J. Mol. Biol., 196:947-950 (1987) and just behind the first 26 nucleotides of the BTG-2 gene (the initiation codon for translation "ATG" is underlined)

The 3' primer has the sequence 5' CAGTGGTACCATACATTT TCTTTTTTTTAGTTAGCCAT 3' (SEQ ID No. 6) and contains the cleavage site for the restriction endonuclease Asp718 and 25 nucleotides complementary to the 3' non-translated sequence of the BTG-2 gene. The amplified sequences are isolated from a 1% agarose gel using a commercially available kit ("Geneclean," BIO 101 Inc., La Jolla, a.). The fragment is then digested with the endonucleases Bam HI and Asp718 and purified again on a 1% agarose gel. This fragment is designated F2.

The vector pRG1 (modification of pVL941 vector, discussed below) is used for the expression of the BTG-2 protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhidrosis virus (AcMNPV) followed by the recognition sites for the restriction endonucleases. The polyadenylation site of the simian virus (SV)40 is used for efficient polyadenylation. For an easy selection of recombinant viruses the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of co-transfected wild-type viral DNA. Many other baculovirus vectors could be used in place of pRG1 such as pAc373, pVL941 and pAcIMl (Luckow, V.A. and Summers, M.D., Virology, 170:31-39) .

The plasmid is digested with the restriction enzymes Bam HI and Asp718 and dephosphorylated using calf intestinal phosphatase by procedures known in the art. The DNA is then isolated from a 1% agarose gel using the commercially available kit ("Geneclean" BIO 101 Inc., La Jolla, Ca.). This vector DNA is designated V2.

Fragment F2 and the dephosphorylated plasmid V2 are ligated with T4 DNA ligase. E.coli HB101 cells are then transformed and bacteria identified that contained the plasmid (pBac BTG-2) with the BTG-2 gene using the respective restriction enzymes. The sequence of the cloned fragment is confirmed by DNA sequencing.

5 µg of the plasmid pBac BTG-2 is co-transfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBac BTG-2 are mixed in a sterile well of a microtiter plate containing 50 µl of serum free Grace's medium (Life Technologies Inc., Gaithersburg, MD). Afterwards 10 µl Lipofectin plus 90 µl Grace's medium are added, mixed and incubated for 15 minutes at room temperature. Then the transfection mixture is added drop-wise to the Sf9 insect cells (ATCC CRL 1711) seeded in a 35 mm tissue culture plate with 1ml Grace' medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation continued at 27°C for four days.

After four days the supernatant is collected and a plaque assay performed similar as described by Summers and Smith (supra). As a modification an agarose gel with "Blue Gal" (Life Technologies Inc., Gaithersburg) is used which allows an easy isolation of blue stained plaques. (A detailed description of a "plaque assay" can also be found in the user's guide for insect cell culture and baculovirology distributed by Life Technologies Inc., Gaithersburg, page 9-10) .

Four days after the serial dilution the viruses are added to the cells and blue stained plaques are picked with the tip of an Eppendorf pipette. The agar containing the recombinant viruses is then resuspended in an Eppendorf tube containing 200 µl of Grace's medium. The agar is removed by a brief centrifugation and the supernatant containing the recombinant baculovirus is used to infect Sf9 cells seeded in 35 mm dishes. Four days later the supernatants of these culture dishes are harvested and then stored at 4°C.

Sf9 cells are grown in Grace's medium supplemented with 10% heat-inactivated FBS. The cells are infected with the recombinant baculovirus V-BTG-2 at a multiplicity of infection (MOI) of 2. Six hours later the medium is removed and replaced with SF900 II medium minus methionine and cysteine (Life Technologies Inc., Gaithersburg). 42 hours later 5 µCi of ³⁵S-methionine and 5 µCi ³⁵S cysteine (Amersham) are added. The cells are further incubated for 16 hours before they are harvested by. centrifugation and the labelled proteins visualized by SDS-PAGE and autoradiography.

### Example 2

### Cloning and expression of BTG-3 using the baculovirus expression system

The DNA sequence encoding the full length BTG-3 protein, ATCC # 97010, is amplified using PCR oligonucleotide. primers corresponding to the 5' and 3' end sequences of the gene:

The 5' primer has the sequence 5' CAGTGGATCCGCCACCATGCAG CTAGAGATCAAAGTGGCCC 3' (SEQ ID No. 7) and contains a Bam HI restriction enzyme site (in bold) followed by 6 nucleotides resembling an efficient signal for the initiation of translation in eukaryotic cells (Kozak, M., J. Mol. Biol., 196:947-950 (1987) and just behind the first 228 nucleotides of the BTG-3 gene (the initiation codon for translation "ATG" is underlined).

The 3' primer has the sequence 5' CAGTGGTACCACGGGCCA GGTAGATGGTCAGTTGGCCAGCAC 3' (SEQ ID No. 8) and contains the cleavage site for the restriction endonuclease Asp718 and 25 nucleotides complementary to the 3' non-translated sequence of the BTG-3 gene. The amplified sequences are isolated from a 1% agarose gel using a commercially available kit ("Geneclean," BIO 101 Inc., La Jolla, Ca.). The fragment is then digested with the endonucleases Bam HI and Asp718 and purified again on a 1% agarose gel. This fragment is designated F2.

The vector pRG1 (modification of pVL941 vector, discussed below) is used for the expression of the BTG-3 protein using the baculovirus expression system (for review see: Summers, M.D. and Smith, G.E. 1987, A manual of methods for baculovirus vectors and insect cell culture procedures, Texas Agricultural Experimental Station Bulletin No. 1555). This expression vector contains the strong polyhedrin promoter of the Autographa californica nuclear polyhedrosis virus (AcMNPV) followed by. the recognition sites for the restriction endonucleases Bam HI and Asp718. The polyadenylation site of the simian virus (SV)40 is used for efficient polyadenylation. For an easy selection of recombinant viruses the beta-galactosidase gene from E.coli is inserted in the same orientation as the polyhedrin promoter followed by the polyadenylation signal of the polyhedrin gene. The polyhedrin sequences are flanked at both sides by viral sequences for the cell-mediated homologous recombination of co-transfected wild-type viral DNA. Many other baculovirus vectors could be used in place of pRG1 such as pAc373, pVL941 and pAcIM1 (Luckow, V.A. and Summers, M.D., Virology, 170:31-39).

The plasmid is digested with the respective restriction enzymes and dephosphorylated using calf intestinal phosphatase by procedures known in the art. The DNA is then isolated from a 1% agarose gel using the commercially available kit ("Geneclean" BIO 101 Inc., La Jolla, Ca.). This vector DNA is designated V2.

Fragment F2 and the dephosphorylated plasmid V2 are ligated with T4 DNA ligase. E.coli HB101 cells are then transformed and bacteria identified that contained the plasmid (pBac BTG-3) with the BTG-3 gene using the enzymes Bam HI and Asp718. The sequence of the cloned fragment is confirmed by DNA sequencing.

5 µg of the plasmid pBac BTG-3 is co-transfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBac BTG-3 are mixed in a sterile well of a microtiter plate containing 50 µl of serum free Grace's medium (Life Technologies Inc., Gaithersburg; MD). Afterwards 10 µl Lipofectin plus 90 µl Grace's medium are added, mixed and incubated for 15 minutes at room temperature. Then the transfection mixture is added drop-wise to the Sf9 insect cells (ATCC CRL 1711) seeded in a 35 mm tissue culture plate with 1ml Grace' medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation continued at 27°C for four days.

After four days the supernatant is collected and a plaque assay performed similar as described by Summers and Smith (supra) . As a modification an agarose gel with "Blue Gal" (Life Technologies Inc., Gaithersburg) is used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution the viruses are added to the cells and blue stained plaques are picked with the tip of an Eppendorf pipette. The agar containing the recombinant viruses is then resuspended in an Eppendorf tube containing 200 µl of Grace's medium. The agar is removed by a brief centrifugation and the supernatant containing the recombinant baculovirus is used to infect Sf9 cells seeded in 35 mm dishes. Four days later the supernatants of these culture dishes are harvested and then stored at 4°C.

Sf9 cells are grown in Grace's medium supplemented with 10% heat-inactivated FBS. The cells are infected with the recombinant baculovirus V-BTG-3 at a multiplicity of infection (MOI) of 2. Six hours later the medium is removed and replaced with SF900 II medium minus methionine and cysteine (Life Technologies Inc., Gaithersburg). 42 hours later 5 µCi of ³⁵S-methionine and 5 µCi ³⁵S cysteine (Amersham) are added. The cells are further incubated for 16 hours before they are harvested by centrifugation and the labelled proteins visualized by SDS-PAGE and autoradiography.

### Example 3

### Expression of Recombinant BTG-2 in COS cells

The expression of plasmid, BTG-2 HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire BTG-2 precursor and a HA tag fused in frame to its 3' end is cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37, 767). The fusion of HA tag to the target protein allows easy detection of the recombinant protein with an antibody that recognizes the HA epitope.

The plasmid construction strategy is described as follows:

The DNA sequence encoding BTG-2, ATCC # 97025, is constructed by PCR using two primers: the 5' primer 5' GGCCC AAGCTTGCCGCCATGCAGCTTGAAATCCAAGTAG 3' (SEQ ID No. 9) contains a Hind III site followed by 23 nucleotides of BTG-2 coding sequence starting from the initiation codon; the 3' sequence 5' ATCGTCTAGATTAGTTAGCCATAACAGGCTGGAATTGCTGGTTAGAATACTGCATG TTATTTAAGCTAAAATTCAAGCCATCTA 3' (SEQ ID No. 10) contains complementary sequences to XbaI site, translation stop codon, HA tag and the last 68 nucleotides of the BTG-2 coding sequence (not including the stop codon). Therefore, the PCR product contains a Hind III site, BTG-2 coding sequence followed by HA tag fused in frame, a translation termination stop codon next to the HA tag, and an XbaI site. The PCR amplified DNA fragment and the vector, pcDNAI/Amp, are digested with Hind III and XbaI restriction enzyme and ligated. The ligation mixture is transformed into E. coli strain SURE (Stratagene Cloning Systems, La Jolla, CA) the transformed culture is plated on ampicillin media plates and resistant colonies are selected. Plasmid DNA is isolated from transformants and examined by restriction analysis for the presence of the correct fragment. For expression of the recombinant BTG-2, COS cells are transfected with the expression vector by DEAE-DEXTRAN method (J. Sambrook, E. Fritsch, T. Maniatis, Molecular Cloning: A Laboratory Manual, Cold Spring Laboratory Press, (1989)). The expression of the BTG-2 HA protein is detected by radiolabelling and immunoprecipitation method (E. Harlow, D. Lane, Antibodies: A Laboratory Manual, Cold Spring Harbor Laboratory Press, (1988)). Cells are labelled for 8 hours with ³⁵S-cysteine two days post transfection. Culture media are then collected and cells are lysed with detergent (RIPA buffer (150 mM NaCl, 1% NP-40, 0.1% SDS, 1% NP-40, 0.5% DOC, 50mM Tris, pH 7.5) . (Wilson, I. et al., Id. 37:767 (1984)). Both cell lysate and culture media are precipitated with a HA specific monoclonal antibody. Proteins precipitated are analyzed on 15% SDS-PAGE gels.

### Example 4

### Expression of Recombinant BTG-3 in COS cells

The expression of plasmid, BTG-3 HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire BTG-3 precursor and a HA tag fused in frame to its 3' end is cloned into the polylinker region of the vector, therefore, the recombinant protein expression is directed under the CMV promoter. The HA tag corresponds to an epitope derived from the influenza hemagglutinin protein as previously described (I. Wilson, H. Niman, R. Heighten, A Cherenson, M. Connolly, and R. Lerner, 1984, Cell 37, 767). The infusion of HA tag to the target protein allows easy detection of the recombinant protein with an antibody that recognizes the HA epitope.

The plasmid construction strategy is described as follows:

The DNA sequence encoding BTG-3, ATCC # 97010, is constructed by PCR using two primers: the 5' primer 5' GGCCC AAGCTTGCCGCCATGCAGCTAGAGATCAAAGTGGC 3' (SEQ ID No. 11) contains a Hind III site followed by 23 nucleotides of BTG-3 coding sequence starting from the initiation codon; the 3' sequence 5' ATCGTCTAGATCAAGCGTAGTCTGGGACGTCGTATGGGTAGGTTG TAGCTGAGGCCTTCCACAAAGGGTGTCTTCTCCAGG 3' (SEQ ID No. 12) contains complementary sequences to an XbaI site, translation stop codon, HA tag and the last 68 nucleotides of the BTG-3 coding sequence (not including the stop codon). Therefore, the PCR product contains a Hind III site, BTG-3 coding sequence followed by HA tag fused in frame, a translation termination stop codon next to the HA tag, and an XbaI site. The PCR amplified DNA fragment and the vector, pcDNAI/Amp, are digested with Hind III and XbaI restriction enzymes and ligated. The ligation mixture is transformed into E. coli strain SURE (Stratagene Cloning Systems, La Jolla, CA) the transformed culture is plated on ampicillin media plates and resistant colonies are selected. Plasmid DNA is isolated from transformants and examined by restriction analysis for the presence of the correct fragment. For expression of the recombinant BTG-3, COS cells are transfected with the expression vector by DEAE-DEXTRAN method (J. Sambrook, E. Fritsch, T. Maniatis, Molecular Cloning: A Laboratory Manual, Cold Spring Laboratory Press, (1989)). The expression of the BTG-3 HA protein is detected by radiolabelling and immunoprecipitation method (E. Harlow, D. Lane, Antibodies: A Laboratory Manual, Cold Spring Harbor Laboratory Press, (1988)). Cells are labelled for 8 hours with ³⁵S-cysteine two days post transfection. Culture media is then collected and cells are lysed with detergent (RIPA buffer (150 mM NaCl, 1% NP-40, 0.1% SDS, 1% NP-40, 0.5% DOC, 50mM Tris, pH 7.5) (Wilson, I. et al., Id. 37:767 (1984)). Both cell lysate and culture media are precipitated with a HA specific monoclonal antibody. Proteins precipitated are analyzed on 15% SDS-PAGE gels.

### Example 5

### Bacterial Expression and Purification of BTG-2

The DNA sequence encoding BTG-2, ATCC # 97025, is initially amplified using PCR oligonucleotide primers corresponding to the 5' and 3' end sequences of the processed BTG-2 gene (minus the signal peptide sequence) and the vector sequences 3' to the BTG-2 gene. Additional nucleotides corresponding to NcoI and Bgl II restriction enzyme sites were added to the 5' and 3' end sequences respectively. The 5' oligonucleotide primer has the sequence ATCGCCATGGGACAGCTT GAAATCCAAGTAGCACTA 3' (SEQ ID No. 13) contains a Nco I restriction enzyme site followed by 24 nucleotides of BTG-2 coding sequence starting from the presumed terminal amino acid of the processed protein codon. The 3' sequence 5' ATC GAGATCTTTAGTTAGCCATAACAGGCTGGAAT 3' (SEQ ID No. 14) contains complementary sequences to a Bgl II restriction site and the last 21 nucleotides of BTG-2. The restriction enzyme sites correspond to the restriction enzyme sites on the bacterial expression vector pQE-60 (Qiagen, Inc. Chatsworth, CA). pQE-60 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-60 is then digested with Nco I and Bgl II. The amplified sequences are ligated into pQE-60 and are inserted in frame with the sequence encoding for the histidine tag and the RBS. The ligation mixture is then used to transform E. coli strain M15/rep 4 (Qiagen, Inc.) by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan^{r}). Transformants are identified by their ability to grow on LB plates and ampicillin/kanamycin resistant colonies are selected. Plasmid DNA is isolated and confirmed by restriction analysis. Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl. After clarification, solubilized BTG-2 is purified from this solution by chromatography on a Nickel-Chelate column under conditions that allow for tight binding by proteins containing the 6-His tag (Hochuli, E. et al., J. Chromatography 411:177-184 (1984)). BTG-2 (95% pure) is eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized). After incubation in this solution for 12 hours the protein is dialyzed to 10 mmolar sodium phosphate.

### Example 6

### Bacterial Expression and Purification of BTG-3

The DNA sequence encoding BTG-3, ATCC # 97010 is initially amplified using PCR oligonucleotide primers corresponding to the 5' and 3' end sequences of the processed BTG-3 gene. Additional nucleotides corresponding to NcoI and Bgl II restriction enzyme sites are added to the 5' and 3' sequences respectively. The 5' oligonucleotide primer has the sequence 5' ATCGCCATGGGACAGCTAGAGATCAAAGTGGCCCTG 3' (SEQ ID No. 15) contains an NcoI restriction enzyme site followed by 24 nucleotides of BTG-3 coding sequence starting from the presumed terminal amino acid of the processed protein. The 3' sequence 5' ATCGAGATCTGTTGGCCAGCACCACGGGCTGG 3' (SEQ ID No. 16) contains complementary sequences to a Bgl II restriction site and the last 21 nucleotides of BTG-3 coding sequence. The restriction enzyme sites correspond to the restriction enzyme sites on the bacterial expression vector pQE-60 (Qiagen, Inc. Chatsworth, CA). pQE-60 encodes antibiotic resistance (Amp^{r}), a bacterial origin of replication (ori), an IPTG-regulatable promoter operator (P/O), a ribosome binding site (RBS), a 6-His tag and restriction enzyme sites. pQE-60 is then digested with Nco I and Bgl II. The amplified sequences are ligated into pQE-60 and are inserted in frame with the sequence encoding for the histidine tag and the RBS. The ligation mixture is then used to transform E. coli strain M15/rep 4 (Qiagen, Inc.) by the procedure described in Sambrook, J. et al., Molecular Cloning: A Laboratory Manual, Cold Spring Laboratory Press, (160860). M15/rep4 contains multiple copies of the plasmid pREP4, which expresses the lacI repressor and also confers kanamycin resistance (Kan^{t}). Transformants are identified by their ability to grow on LB plates and ampicillin/kanamycin resistant colonies are selected. Plasmid DNA is isolated and confirmed by restriction analysis. Clones containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl. After clarification, solubilized BTG-3 is purified from this solution by chromatography on a Nickel-Chelate column under conditions that allow for tight binding by proteins containing the 6-His tag (Hochuli, E. et al., J. Chromatography 411:177-184 (16084)). BTG-3 (95 % pure) is eluted from the column in 6 molar guanidine HCl pH 5.0 and for the purpose of renaturation adjusted to 3 molar guanidine HCl, 100mM sodium phosphate, 10 mmolar glutathione (reduced) and 2 mmolar glutathione (oxidized). After incubation in this solution for 12 hours the protein is dialyzed to 10 mmolar sodium phosphate.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in SEQ ID No. 4;
(b) polynucleotides having the coding sequence as shown in SEQ ID No. 3 encoding at least the mature form of the polypeptide;
(c) polynucleotides encoding the polypeptide having the amino acid sequence of at least the mature form of the polypeptide encoded by the cDNA contained in ATCC 97010;
(d) polynucleotides having the coding sequence of the cDNA contained in ATCC 97010 encoding at least the mature form of the polypeptide;
(e) polynucleotides encoding a fragment, analog or derivative of a polypeptide encoded by a polynucleotide of any one of (a) to (d); and
(f) polynucleotides the complementary strand of which hybridizes under stringent conditions to a polynucleotide as defined in any one of (a) to (e) and which encode a polypeptide having BTG-3 antiproliferative activity
or the complementary strand of such a polynucleotide.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the vector of claim 5 or 6.

8. A process for producing a polypeptide having BTG-3 antiproliferative activity comprising: culturing the host cell of claim 7 and recovering the polypeptide encoded by said polynucleotide from the culture.

9. A process for producing cells capable of expressing a polypeptide having BTG-3 antiproliferantive activity comprising genetically engineering cells with the vector of claim 5 or 6.

10. A polypeptide having the amino acid sequence encoded by a polynucleotide of any one of claims 1 to 4 or obtainable by the process of claim 8.

11. An antibody against the polypeptide of claim 10.

12. A nucleic acid molecule which specifically hybridizes to a polynucleotide of any one of claims 1 to 4.

13. An agonist of the polypeptide of claim 10.

14. An antagonist/inhibitor against the polypeptide of claim 10.

15. A pharmaceutical composition comprising the polynucleotide of any one of claims 1 to 4, the polypeptide of claim 10 or a DNA encoding and capable of expressing said polypeptide in vivo, or the agonist of claim 13 or the antagonist of claim 14 and optionally a pharmaceutically acceptable carrier.

16. A diagnostic composition comprising the polynucleotide of any one of claims 1 to 4, the nucleic acid molecule of claim 12 or the antibody of claim 11.

17. Use of the polypeptide of claim 10, of the polynucleotide of any one of claims 1 to 4 or the agonist of claim 13 for the preparation of a pharmaceutical composition for the regulation of tumor/growth suppression, for inhibiting angiogenesis of tumors or cancer cell proliferation, for preventing uncontrolled wound healing or for the treatment of restenosis.

18. Use of the antagonist of claim 14 or the nucleic acids molecule of claim 12 for the preparation of a pharmaceutical composition for the treatment of leukemia

19. A process for identifying agonists or antagonists to BTG-3 comprising:
(a) combining a compound to be screened, a labelled polypeptide of claim 10 and a reaction mixture containing cells which express a receptor for BTG-3; and
(b) determining the extent of binding of said polypeptide to the receptors.

20. A process for diagnosing a disease or a susceptibility to a disease related to an altered level of expression of the polypeptide of claim 10 comprising: determining a mutation in the nucleic acid sequence encoding said polypeptide.

21. A diagnostic process comprising: analyzing for the presence of the polypeptide of claim 10 in a sample derived from a host.


SEQUENCE LISTING
(1) GENERAL INFORMATION:
(i) APPLICANT: KUNSCH, ET AL.

(ii) TITLE OF INVENTION: Human B-Cell Translocation Genes-2 and 3

(iii) NUMBER OF SEQUENCES: 16

(iv) CORRESPONDENCE ADDRESS:

(A) ADDRESSEE: CARELLA, BYRNE, BAIN, GILFILLAN,
CECCHI, STEWART & OLSTEIN
(B) STREET: 6 BECKER FARM ROAD
(C) CITY: ROSELAND
(D) STATE: NEW JERSEY
(E) COUNTRY: USA
(F) ZIP: 07068

(v) COMPUTER READABLE FORM:
(A) MEDIUM TYPE: 3.5 INCH DISKETTE
(B) COMPUTER: IBM PS/2
(C) OPERATING SYSTEM: MS-DOS
(D) SOFTWARE: WORD PERFECT 5.1

(vi) CURRENT APPLICATION DATA:
(A) APPLICATION NUMBER:
(B) FILING DATE: Submitted herewith
(C) CLASSIFICATION:

(vii PRIOR APPLICATION DATA
(A) APPLICATION NUMBER:
(B) FILING DATE:

(viii) ATTORNEY/AGENT INFORMATION:
(A) NAME: FERRARO, GREGORY D.
(B) REGISTRATION NUMBER: 36,134
(C) REFERENCE/DOCKET NUMBER: 325800-262

(ix) TELECOMMUNICATION INFORMATION:
(A) TELEPHONE: 201-994-1700
(B) TELEFAX: 201-994-1744

(2) INFORMATION FOR SEQ ID NO:1:

(i) SEQUENCE CHARACTERISTICS
(A) LENGTH: 1843 BASE PAIRS
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:1:

GGCACGAGAT TTTGTGGCGT AGAGCTATGC AGCTTGAAAT CCAAGTAGCA CTAAATTTTA 60
TTATTTCGTA TTTGTACAAT AAGCTTCCCA GGAGACGTGT CAACATTTTT GGTGAAGAAC 120
TTGAAAGACT TCTTAAGAAG AAATATGAAG GGCACTGGTA TCCTGAAAAG CCATACAAAG 180
GATCGGGGTT TAGATGTATA CACATAGGGG AGAAAGTGGA CCCAGTGATT GAACAAGCAT 240
CCAAAGAGAG TGGTTTGGAC ATTGATGATG TTCGTGGCAA TCTGCCACAG GATCTTAGTG 300
TTTGGATCGA CCCATTTGAG GTTTCTTACC AAATTGGTGA AAAGGGACCA GTGAAGGTGC 360
TTTACGTGGA TGATAATAAT GAAAATGGAT GTGAGTTGGA TAAGGAGATC AAAAACAGCT 420
TTAACCCAGA GGCCCAGGTT TTTATGCCCA TAAGTGACCC AGCCTCATCA GTGTCCAGCT 480

CTCCATCGCC TCCTTTTGGT CACTCTGCTG CTGTAAGCCC TACCTTCATG CCCCGGTCCA 540
CTCAGACTTT AACCTTTACC ACTGCCACTT TTGGCTGCCA CCAAGTTCGG CTCTACCAAA 600
ATGAAGATAG TGGCCGTAGC AACAAGGTTG CACGTACTTC TCCCATCAAC CTCGGCTTGA 660
ATGTGAATGA CCTCTTGAAG CAGAAAGCCA TCTCTTCCTC AATGCACTCT CTGTATGGGC 720
TTGGCTTGGG TAGCCAGCAG CAGCCACAGC AACAGCAGCA GCCAGCCCAG CCGCCACCGC 780
CACCACCACC ACCACAGGAG GAACAANAGC AGAAAACCTC TGCTCTTTCT CCTAATGCCA 840
AGGAATTTAT TTTTCCTAAT ATGCAGGGTC AAGGTAGTAG TACCAATGGA ATGTTCCCAG 900
GTGACAGCCC CCTTAACCTC AGTCCTCTCC AGTACAGTAA TGCCTTTGAT GTGTTTGCAG 960
CCTATGGAGG CCTCAATGAG AAGTCTTTTG TAGATGGCTT GAATTTTAGC TTAAATAACA1020
TGCAGTATTC TAACCAGCAA TTCCAGCCTG TTATGGCTAA CTAAAAAAAA GAAAATGTAT1080
CGTACAAGTT AAAATGCACG GGCCAAGGGG GGATTTTTTT TTTCACCTCC TTGAGAATTT1140
TTTTTTTTAA GCTTATAGTA AGGATACATT CAAGCTTGGT TAAAAAAATA ATAATAAAAC1200
ATGCATCATT TTTCATTTGC CAACCAAGCA CAAAGTTATT TTATGCTGCC TGTATATTTT1260
AAAGTATACT CTCAGATATG CCCTCTTACA GTATTTTAAG ATATTAGCAA AGGACATGGC1320
TTGATTTTTT TTTATAAAAA TTGGCACTAA TAAGTGGGTT TATTGGTCTT TTCTAATTGT1380
ATAATTTAAT TTAGTACCAA AGTTTGTAAA ATATCAGAGG ATATATATAT ATTGTATCCT1440
ACGACATGGT ATTGCATTTA TATCTTTTTA CTACAGTGAT CTGTGACAGC AGCAGCCTCA1500
TGTTGTATTT TTTTTACTGA AATTGTAAAA TATCCATCTT AAAGACATCA ACTATTCTAA1560
AAATTGTGTA CAGGATATTC CTTTAGTGGT GGAATTAAAA TGTGCGAATA CTTGCTTTCT1620
CCAAAAAAAT GTATTTTCTG TTAAAAGTTT AAAGATTTTT GCTATATATT ATGGAAGGAA1680
AATGTAATCG TAAATATTAA TTTTGTACCT ATATTGTGCA ATACTTGAAA AAAACGGTAT1740
AAAAGTATTT TGAGTCAGTG TCTTACATGT TAAGAGGGAC TGAAATAGTT TATATTAAGT1800
TTGTATTAAA ATTCTTTAAA ATTAAAAAAA AAAAAAAAAA AAA 1843

(2) INFORMATION FOR SEQ ID NO:2:
(i) SEQUENCE CHARACTERISTICS
(A) LENGTH: 345 AMINO ACIDS
(B) TYPE: AMINO ACID
(C) STRANDEDNESS:
(D) TOPOLOGY: LINEAR

(ii) MOLECULE TYPE: PROTEIN

(xi) SEQUENCE DESCRIPTION: SEQ ID NO:2:

Met Gln Leu Glu Ile Gln Val Ala Leu Asn Phe Ile Ile Ser Tyr
-25 -20 -15
Leu Tyr Asn Lys Leu Pro Arg Arg Arg Val Asn Ile Phe Gly Glu
-10 -5 1 5
Glu Leu Glu Arg Leu Leu Lys Lys Lys Tyr Glu Gly His Trp Tyr 10
15 20
Pro Glu Lys Pro Tyr Lys Gly Ser Gly Phe Arg Cys Ile His Ile 25
30 35
Gly Glu Lys Val Asp Pro Val Ile Glu Gln Ala Ser Lys Glu Ser 40
45 50
Gly Leu Asp Ile Asp Asp Val Arg Gly Asn Lys Pro Gln Asp Lys 55
60 65
Ser Val Trp Ile Asp Pro Phe Glu Val Ser Tyr Gln Ile Gly Glu 70
75 80
Lys Gly Pro Val Lys Val Leu Tyr Val Asp Asp Asn Asn Glu Asn 85
90 95
Gly Cys Glu Leu Asp Lys Glu Ile Lys Asn Ser Phe Asn Pro Glu 100
105 110
Ala Gln Val Phe Met Pro Ile Ser Asp Pro Ala Ser Ser Val Ser 115
120 125
Ser Ser Pro Ser Pro Pro Phe Gly His Ser Ala Ala Val Ser Pro 130
135 140
Thr Phe Met Pro Arg Ser Thr Gln Thr Leu Thr Phe Thr Thr Ala 145
150 155
Thr Phe Gly Cys His Gln Val Arg Leu Tyr Gln Asn Glu Asp Ser 160
165 170
Gly Arg Ser Asn Lys Val Ala Arg Thr Ser Pro Ile Asn Leu Gly 175
180 185

Leu Asn Val Asn Asp Leu Leu Lys Gln Lys Ala Ile Ser Ser Ser 190
195 200
Met His Ser Leu Tyr Gly Leu Gly Leu Gly Ser Gln Gln Gln Pro 205
210 215
Gln Gln Gln Gln Gln Pro Ala Gln Pro Pro Pro Pro Pro Pro Pro 220
225 230
Pro Gln Glu Glu Gln Gln Gln Lys Thr Ser Ala Leu Ser Pro Asn 235
240 245
Ala Lys Glu Phe Ile Phe Pro Asn Met Gln Gly Gln Gly Ser Ser 250
255 260
Thr Asn Gly Met Phe Pro Gly Asp Ser Pro Leu Asn Leu Ser Pro 265
270 275
Leu Gln Tyr Ser Asn Ala Phe Asp Val Phe Ala Ala Tyr Gly Gly 280
285 290
Leu Asn Glu Lys Ser Phe Val Asp Gly Leu Asn Phe Ser Leu Asn 295
300 305
Asn Met Gln Tyr Ser Asn Gln Gln Phe Gln Pro Val Met Ala Asn
310 315 320

(2) INFORMATION FOR SEQ ID NO:3:

(i) SEQUENCE CHARACTERISTICS
(A) LENGTH: 1352 BASE PAIRS
(B) TYPE: NUCLEIC ACID
(C) STRANDEDNESS: SINGLE
(D) TOPOLOGY: LINEAR

(ii) MOLECULE TYPE: cDNA

(xi) SEQUENCE DESCRIPTION: SEQ ID NO:3:

GGAATTCGGC ACGAGCAACC CTCAACGACG AAAAGGACTT CGGTCCCCTG GCCCGGCGAC 60
GCCCGGRAAG GAAAGGAGAG CGACCTCCGC CCCGCGCTCA GGCCACCCTG GAGGGAGAAG 120
CCGCCCCGCG GCGGGTAGAG CGCCCCGCCG CCNCGYWAGN ACCCGAAGCC GCCTGGAGCC 180
CAAGGCTGTA CACGTGCCCT GTGCTGAGGC TCTGCCTAGG AAAGGACCAT GCAGCTAGAG 240
ATCAAAGTGG CCCTGAACTT CATCCATCTC CTACTTGTAC AACAAGCTGC CCCGGCGCCC 300
GGGCAGAACC TGTTTGGGGA GGAGCTAGAG CGGCTTTTGA AAAGGAAATA TGAAGGCCAC 360
TGGTACCCTG AGAAGCCACT GAAAGGCTCT GGCTTCCGCT GTGTTCACAT TGGGGAGATG 420
GTGGACCCCG TGGTGGAGCT GGCCGCCAAG CGGAGTGGCC TGGCGGTGGA AGATGTGCGG 480
GCCAATGTGC CTGAGGAGCT GAGTGTCTGG ATTGATCCCT TTGAGGTGTC CTACCAGATT 540
GGTGAGAAGG GAGCTGTGAA AGTGCTGTAC CTGGATGACA GTGAGGGTTG CGGTGCCCCA 600
GAGCTGGACA AGGAGATCAA GAGCAGCTTC AACCCTGACG CCCAGGTGTT CGTGCCCATT 660
GGCAGCCAGG ACAGCTCCCT GTCCAACTCC CCATCGCCAT CCTTTGGCCA GTCACCCAGC 720
CCTACCTTCA TTCCCCGCTC CGCTCAGCCC ATCACCTTCA CCACCGCCTC CTTCGCTGCC 780
ACCAAATTTG GCTCCACTAA GATGAAGAAG GGGGGCGGGG CAGCAAGTGG TGGGGGTGTA 840
GCCAGCAGTG GGGCGGGTGG CCAGCAGCCA CCACAGCAGC CTCGCATGGC CCGCTCACCC 900
ACCAACAGCC TGCTGAAGCA CAAGAGCCTC TCTCTGTCTA TGCATTCACT GAACTTCATC 960
ACGGCCAACC CGGCCCCTCA GTCCCAGCTC TCACCCAATG CCAAGGAGTT CGTGTACAAC1020
GGTGGTGGCT CACCCAGCCT CTTCTTTGAT GCGGCCGATG GCCAGGGCAG CGGCACCCCA1080
GGCCCGTTTG GAGGCAGTGG GGCTGGCACC TGCAACAGCA GCAGCTTTGA CATGGCCCAG1140
GTATTTGGAG GTGGTGCCAA CAGCCTCTTC CTGGAGAAGA CACCCTTTGT GGAAGGCCTC1200
AGCTACAACC TGAACACCAT GCAGTATCCC AGCCAGCAGT TCCAGCCCGT GGTGCTGGCC1260
AACTGACCAT CTACCTGGCC CGTGGGGGCA GGAGCACCCA AGACCACAGA AAAGAGAAAG1320
GAAAGGCCAA AAAAAAAAAA AAAAAACTCG AG 1352

(2) INFORMATION FOR SEQ ID NO:4:
(i) SEQUENCE CHARACTERISTICS
(A) LENGTH: 345 AMINO ACIDS
(B) TYPE: AMINO ACID
(C) STRANDEDNESS:
(D) TOPOLOGY: LINEAR

(ii) MOLECULE TYPE: PROTEIN

(xi) SEQUENCE DESCRIPTION: SEQ ID NO:4:

Met Gln Leu Glu Ile Lys Val Ala Leu Asn Phe Ile His Leu Leu
-25 -20 -15
Leu Val Gln Gln Ala Ala Pro Ala Pro Gly Gln Asn Leu Phe Gly
-10 -5 1 5
Glu Glu Leu Glu Arg Leu Leu Lys Arg Lys Tyr Glu Gly His Trp
10 15 20
Tyr Pro Glu Lys Pro Leu Lys Gly Ser Gly Phe Arg Cys Val His
25 30 35
Ile Gly Glu Met Val Asp Pro Val Val Glu Leu Ala Ala Lys Arg
40 45 50
Ser Gly Leu Ala Val Glu Asp Val Arg Ala Asn Val Pro Glu Glu 55
60 65
Leu Ser Val Trp Ile Asp Pro Phe Glu Val Ser Tyr Gln Ile Gly 70
75 80
Glu Lys Gly Ala Val Lys Val Leu Tyr Leu Asp Asp Ser Glu Gly 85
90 95
Cys Gly Ala Pro Glu Leu Asp Lys Glu Ile Lys Ser Ser Phe Asn 100
105 110
Pro Asp Ala Gln Val Phe Val Pro Ile Gly Ser Gln Asp Ser Ser 115
120 125
Leu Ser Asn Ser Pro Ser Pro Ser Phe Gly Gln Ser Pro Ser Pro 130
135 140
Thr Phe Ile Pro Arg Ser Ala Gln Pro Ile Thr Phe Thr Thr Ala 145
150 155
Ser Phe Ala Ala Thr Lys Phe Gly Ser Thr Lys Met Lys Lys Gly 160
165 170
Gly Gly Ala Ala Ser Gly Gly Gly Val Ala Ser Ser Gly Ala Gly 175
180 185
Gly Gln Gln Pro Pro Gln Gln Pro Arg Met Ala Arg Ser Pro Thr 190
195 200
Asn Ser Leu Leu Lys His Lys Ser Leu Ser Leu Ser Met His Ser 205
210 215
Leu Asn Phe Ile Thr Ala Asn Pro Ala Pro Gln Ser Gln Leu Ser 220
225 230
Pro Asn Ala Lys Glu Phe Val Tyr Asn Gly Gly Gly Ser Pro Ser 235
240 245
Leu Phe Phe Asp Ala Ala Asp Gly Gln Gly Ser Gly Thr Pro Gly 250
255 260
Pro Phe Gly Gly Ser Gly Ala Gly Thr Cys Asn Ser Ser Ser Phe 265
270 275
Asp Met Ala Gln Val Phe Gly Gly Gly Ala Asn Ser Leu Phe Leu 280
285 290
Glu Lys Thr Pro Phe Val Glu Gly Leu Ser Tyr Asn Leu Asn Thr 295
300 305
Met Gln Tyr Pro Ser Gln Gln Phe Gln Pro Val Val Leu Ala Asn
310 315 320

(2) INFORMATION FOR SEQ ID NO:5:

(i) SEQUENCE CHARACTERISTICS
(A) LENGTH: 41 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:5:

CAGTGGATCC GCCACCATGC AGCTTGAAAT CCAAGTAGCA C 41

(2) INFORMATION FOR SEQ ID NO:6:

(i) SEQUENCE CHARACTERISTICS
(A) LENGTH: 38 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:6:

CAGTGGTACC ATACATTTTC TTTTTTTTAG TTAGCCAT 38

(2) INFORMATION FOR SEQ ID NO:7:

(i) SEQUENCE CHARACTERISTICS
(A) LENGTH: 41 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:7:

CAGTGGATCC GCCACCATGC AGCTAGAGAT CAAAGTGGCC C 41

(2) INFORMATION FOR SEQ ID NO:8:

(i) SEQUENCE CHARACTERISTICS
(A) LENGTH: 42 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:8:

CAGTGGTACC ACGGGCCAGG TAGATGGTCA GTTGGCCAGC AC 42

(2) INFORMATION FOR SEQ ID NO:9:

(i) SEQUENCE CHARACTERISTICS
(A) LENGTH: 39 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:9:

GGCCCAAGCT TGCCGCCATG CAGCTTGAAA TCCAAGTAG 39

(2) INFORMATION FOR SEQ ID NO:10:

(i) SEQUENCE CHARACTERISTICS
(A) LENGTH: 84 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:10:

ATCGTCTAGA TTAGTTAGCC ATAACAGGCT GGAATTGCTG GTTAGAATAC TGCATGTTAT 60
TTAAGCTAAA ATTCAAGCCA TCTA 84

(2) INFORMATION FOR SEQ ID NO:11:

(i) SEQUENCE CHARACTERISTICS
(A) LENGTH: 40 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:11:

GGCCCAAGCT TGCCGCCATG CAGCTAGAGA TCAAAGTGGC 40

(2) INFORMATION FOR SEQ ID NO:12:

(i) SEQUENCE CHARACTERISTICS
(A) LENGTH: 81 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:12:

ATCGTCTAGA TCAAGCGTAG TCTGGGACGT CGTATGGGTA GGTTGTAGCT GAGGCCTTCC 60
ACAAAGGGTG TCTTCTCCAG G 81
(2) INFORMATION FOR SEQ ID NO:13:

(i) SEQUENCE CHARACTERISTICS
(A) LENGTH: 36 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:13:

ATCGCCATGG GACAGCTTGA AATCCAAGTA GCACTA 36

(2) INFORMATION FOR SEQ ID NO:14:

(i) SEQUENCE CHARACTERISTICS
(A) LENGTH: 35 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:14:

ATCGAGATCT TTAGTTAGCC ATAACAGGCT GGAAT 35

(2) INFORMATION FOR SEQ ID NO:15:

(i) SEQUENCE CHARACTERISTICS
(A) LENGTH: 36 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:15:
ATCGCCATGG GACAGCTAGA GATCAAAGTG GCCCTG 36

(2) INFORMATION FOR SEQ ID NO:16:

(i) SEQUENCE CHARACTERISTICS
(A) LENGTH: 32 BASE PAIRS
(B) TYPE: NUCLEIC ACID
(C) STRANDEDNESS: SINGLE
(D) TOPOLOGY: LINEAR

(ii) MOLECULE TYPE: Oligonucleotide

(xi) SEQUENCE DESCRIPTION: SEQ ID NO:16:

ATCGAGATCT GTTGGCCAGC ACCACGGGCT GG 32